# EUROPEAN PATENT APPLICATION

(11) **EP 4 501 946 A1**
(43) Date of publication of application: **05.02.2025**
(21) Application number: 23188672.2
(22) Date of filing: 31.07.2023
(51) Int. Cl.: C07K 14/005, C12N 15/86

(54) **SELF-AMPLIFYING BIOLOGICS FOR TARGETED THERAPY**

(71) Applicant: LoopLab Bio GmbH, 1190 Vienna (AT)
(72) Inventor: STRITZKER, Jochen, 3400 Kierling (AT); TSCHISMAROV, Roland, 1200 Vienna (AT); SCHRAUF, Sabrina, 7072 MÖRBISCH (AT); BATTIN, Claire, 1040 Vienna (AT); DE SOUSA-LINHARES, Annika, 3452 Michelndorf (AT); KONSTANTOULAS, Constantine, 1070 Vienna (AT); NEUBAUER, Simone, 1110 Vienna (AT); TAUBER, Erich, 3426 Muckendorf (AT)
(74) Representative: Eisenführ Speiser

(57) **Abstract**

The present invention relates to a self-amplifying biologic (SelfAmp) in the form of a self-replicating particle in a biological host for transport of a therapeutic payload to a target cell. Further, the present invention relates to at least one vector encoding a SelfAmp. The present invention also relates to a method for the production of a Self-Amp and use thereof in a pharmaceutical composition comprising a SelfAmp for use in a method for a treatment of a disease. Further, the present invention relates to a kit comprising a SelfAmp.

## Description

### Technical Field

The present invention relates to a self-amplifying biologic (SelfAmp) in the form of a self-replicating particle in a biological host for transport of a therapeutic payload to a target cell. Further, the present invention relates to at least one vector encoding a SelfAmp. The present invention also relates to a method for the production of a Self-Amp and use thereof in a pharmaceutical composition comprising a SelfAmp for use in a method for a treatment of a disease. Further, the present invention relates to a kit comprising a SelfAmp.

### Background of the Invention

### Introduction

Biotechnological advancements in the field of targeted drug delivery hold immense potential to transform medical treatment paradigms.

Protein-based therapies offer a high level of target specificity, but are limited by their inability to amplify within the body, which results in limited durability. Furthermore, they are prone to non-specific interactions with non-target structures.

RNA-based approaches currently face efficiency challenges, particularly when utilizing lipid nanoparticle targeting. These methods are also constrained by a limited payload capacity and non-adjustable replication rates in self-replicating RNA. The inherent immune stimulation these approaches trigger is not adequately adjustable, and modulated immunogenicity, associated with modified bases, is incompatible with self-replicating RNA. Cell-based therapies, while promising, present complex and expensive manufacturing challenges due to the need for ex vivo cultivation. Their success is contingent upon isolating specific cell types or differentiating stem cells. The unique MHC-haplotype of each patient adds an extra layer of complexity, necessitating a careful balance between personalized treatments and readily available off-the-shelf products.

In contrast, the utilization of viruses with their inherent ability to efficiently infiltrate cells for drug delivery has emerged as one of the most promising approaches, as the specificity of viruses can be manipulated to target distinct cell types, enabling precision therapy. This selective action minimizes off-target interactions, thus heightening the safety and therapeutic efficacy of the treatment.

While the utilization of viruses as a drug delivery system has demonstrated remarkable efficacy, especially in gene therapy, this method also faces several significant challenges that temper their application.

A primary concern lies in the infectivity and immunogenicity of viruses. When introduced into the body, viruses - if not appropriately attenuated or inactivated - can elicit a sometime severe immune response, which can lead to their neutralization and possible induction of deleterious inflammatory responses. This immune recognition limits the therapeutic effectiveness of the virus-based drug delivery system and may even give rise to systemic complications.

Another notable limitation is the cargo capacity of most viruses. The encapsulation of therapeutic genes is constrained by the size capacity of the viral vector, which may restrict the introduction of larger therapeutic genes or the simultaneous delivery of multiple genes.

For decades now, researchers have been making use of biotechnology to modify viruses for a tailored deployment in a variety of diseases by using a reverse genetics approach, efficiently creating recombinant viruses by constructing a full-length cDNA copy of the viral genome. This copy is then inserted into cells, where it is transcribed and replicated, resulting in the production of a modified virus. By altering the cDNA sequence, scientists can engineer viruses with specific properties, such as inserting reporter genes, removing virulence factors, or swapping protein genes from related viruses. The modified viruses are grown in cells, harvested, purified, and quantified for further applications. This technique has significantly advanced our knowledge of viral pathogenesis, replication, and host interactions, and has also aided in the development of oncolytic viruses and new vaccines (STOBART, CS and MOORE, ML, RNA virus reverse genetics and vaccine design, Viruses 6, 2531-2550 (2014)).

Vaccination, a robust and effective safeguard against infectious diseases, has seen significant advancement since its inception. Live attenuated viruses (LAVs), engineered to induce minimal or no disease, have been instrumental in the battle against such health threats.

For example, measles virus (MV), a member of the Morbillivirus genus in the Paramyxoviridae family, has been widely used for vaccination. The enveloped virus features a non-segmented RNA genome of negative polarity, spanning 15,894 base pairs. Notably, this virus can only induce measles once, as exposure triggers a robust immune response, culminating in lifelong immunity against the disease.

This remarkable trait is capitalized upon in the measles vaccine, one of many LAVs approved by the World Health Organization, which includes vaccines against diseases like polio, rotavirus, and yellow fever. However, it's important to note that despite their benefits, LAVs may raise concerns regarding safety and stability. Potential issues could include reversion, purity, and possible contamination within the viral preparations used for vaccines and other treatments.

The measles virus (MV) vaccine originates from the Edmonston strain of the virus, isolated in 1954 and subsequently attenuated through adaptation to chicken embryonic fibroblasts and further passaging. The resulting Schwarz and Moraten strains have since become the most widely used measles vaccines, providing lifelong immunity with one or two doses. These vaccines are cost-effective, safe, and have been administered successfully to millions of children globally, mitigating the burden of measles.

In an intriguing development, outlined in patent EP 1 939 214 B1, the inventors expanded upon the measles vaccine's potential, harnessing it as a vector capable of stably expressing various genes or large gene combinations. This involved modifying the Schwarz MV measles vaccine and integrating additional transcriptional units (ATUs) at various points within the viral genome. The ATUs contained multi-cloning site cassettes inserted into a copy of the intergenic N-P region of the viral genome, which bears the cis-acting sequences essential for transcription.

The measles vaccine, beyond protecting against measles itself, has therefore been recognized for its potential utility in the broader field of immunization. This includes its deployment as a viral vector for the expression of foreign genes and the development of vaccines against other diseases, revealing the untapped potential of existing vaccination tools.

In recent years, there have been advances in developing measles virus-based vaccines for other diseases, leveraging the safety and efficacy record of the measles vaccine. With the advent of recombinant DNA technologies, vaccine strain-derived MVs can now encode and express additional foreign antigens during transient virus replication following immunization, inducing effective humoral and cellular immune responses against both the MV vector and the foreign antigen cargo (EBENING, A. et al., Versatility of live-attenuated measles viruses as platform technology for recombinant vaccines, Npj Vaccines 119, 1-12 (2022)). This includes the reutilization of measles virus-based vaccine vectors for diseases like HIV, dengue, and SARS-CoV-2 and its modification for targeted cancer therapy.

However, the efficient production of engineered viruses presents many technical challenges, a significant portion of which stem from the organization of the viral genome itself.

Among these viruses, those categorized in the order Mononegavirales (comprising the family of Paramyxoviridae) have non-segmented, negative-stranded RNA genomes with genes arrayed in a remarkably conserved sequence. These mononegaviral genes, or transcriptional units, are aligned sequentially and typically interrupted by non-transcribed intergenic sequences. Viral RNA-dependent RNA polymerase engages the single promoter at the 3' end of the genome, sequentially transcribing genes towards the 5' end by identifying gene start (GS) and gene end (GE) sequences at each gene boundary. A distinct gradient in gene expression, termed 'polar attenuation', emerges due to imperfect reinitiation of transcription at each gene boundary, leading to progressively less abundant mRNA levels for downstream genes.

Advancements in reverse genetics have positioned Paramyxoviruses as valuable tools for gene expression vectors. As described above, the optimal expression levels of foreign genes are typically achieved when the transcriptional units are inserted at the 3'-proximal first locus of the viral genome. However, this insertion often impairs virus multiplication, particularly when the 3'-proximal locus is utilized. While inserting the foreign gene into more distant positions can mitigate this effect on virus multiplication, it consequently diminishes the expression level of the inserted genes. The length of the genome also presents a limiting factor for Paramyxovirus-derived vectors, as longer gene insertions usually reduce virus replication more significantly.

Takeda et al. (2006) demonstrated the potential of recombinant paramyxoviruses with segmented RNA genomes by generating a Measles virus (MV) with a segmented RNA genome using a highly efficient reverse genetics system. The viruses were viable and replicated in cultured cells, yielding three-segmented MVs that could accommodate up to six additional transcriptional units (TAKEDA, M et al., Generation of measles virus with a segmented RNA genome, Journal of Virology 80, 9, 4242-4248 (2006)). They proved how segmentation as a biotechnological tool could theoretically overcome limitations in viral genome length and that the impact of polar attenuation in transcription might be reduced. However, the authors only tested the efficacy of segmented measles in cell culture, still lacking clinical trials.

Gao et al. (2008) tried to express transgenes from Newcastle Disease Virus (NDV) with a segmented genome. The authors of this study generated a recombinant NDV possessing a two-segmented genome, which allowed for efficient replication and transcription. Each genomic segment was flanked by NDV type 3' and 5' noncoding termini, which enables the virus to stably grow in embryonated chicken eggs over multiple passages, utilizing fluorescent reporter genes (GAO, Q et al., Expression of transgenes from Newcaste Disease Virus with a segmented genome, Journal of Virology 82, No. 6, 2692-2698 (2008)). But questions arise regarding the stability and immune response elicited by foreign proteins in such methodologies (HU, Z. et al., Newcaste Disease Virus as a vaccine vector for 20 years: a focus on maternally derived antibody interference, Vaccines 8, 222, 1-24 (2020)).

Miest et al. (2011) established a murine model with the purpose of defining a cancer therapy and vaccination. To this end, they substituted the complete H and F surface glycoproteins of the measles virus with those from the Canine morbillivirus (former Canine distemper virus, CDV). CDV is a morbillivirus closely related to MeV but is resistant to neutralization by measles-immune sera. Additionally, a single-chain variable fragment (scFv) was fused to the C-terminus of the CDV H-protein to target specific receptors on cancer cells, which enhanced oncolytic efficacy with a prodrug convertase gene. The chimeric virus - as a proof-of-principle of an oncolytic virus delivering a chemotherapeutic prodrug - showed resistance to neutralization by both mice and human sera (MIEST, TS et al., Envelope-chimeric entry-targeted measles virus escapes neutralization and achieves oncolysis, Molecular Therapy 19, 10, 1813-1820 (2011)).

However, while pronounced fusogenicity and syncytia-formation are advantageous in anticancer therapies, they present a substantial challenge when the targeted tissue is meant to be preserved, rather than destroyed, as required in treatments aiming for a gentler therapeutic intervention.

Munoz-Alia et al. (2021) addressed the prevalence of measles seropositivity by engineering a vaccine-lineage chimeric MV-CDV virus and retargeting it for oncolytic virotherapy. It could be shown that the MV-CDV chimera can target and destroy CD46-expressing cancer cells in mouse models (MUNOZ-ALIA, MA et al., MeV-Stealth: a CD46-specific oncolytic measles virus resistant to neutralization by measles-immune human serum, PLoS Pathog. 17, 2, e1009283 (2021)). However, the constructs employed were highly aggressive, eradicating a majority of target cells through intense cell-cell fusion activity. This process can cause harm and provoke inflammation in the adjacent tissue, potentially causing unintentional damage to healthy tissue. Therefore, for well-rounded immunotherapy, there is an urgent need for more advanced MV-chimeras to offer flexible and safe therapeutic solutions.

The above mentioned research focuses on oncolytic viral therapies and thus only targets receptors abundant on tumor cells. By using non-segmented viral constructs it is not taken advantage of comparably higher expression levels of viral and payload proteins of segmented viral constructs. Additionally, the potential to achieve increased host immune-tolerance by using modified CDV vaccine viral strain as a basis for the CDV-chimeric surface proteins instead of the wildtype strain remains unexploited.

In general, the employment of chimeric F- and H-proteins can lead to more efficient evasion of the immune system. Integration of virus proteins from various virus species into a synthetic chimeric virus seems to be a feasible strategy. This involves substituting the F- and H-proteins on the Measles Virus (MV) envelope with structurally akin yet immunologically inert glycoproteins from a related animal virus, which could potentially allow the therapeutic virus to bypass immune system detection and neutralization.

In addition to leveraging the F- and H-proteins to facilitate fusion and receptor binding activities of the viral delivery system, the use of a single fusion protein, such as the rhabdovirus G-protein, has been explored. The rhabdoviral G-protein exhibits a broad cellular tropism, enabling cellular entry into a wide array of cell types. This advantage has made it a valuable tool in lentivirus-based gene therapy. Beyond these established applications, researchers have also attempted to alter the tropism of a measles virus (MV) by substituting its envelope proteins with the vesicular stomatitis virus (VSV) G-protein, a process known as pseudotyping. However, the viral titers were found to be up to 50 times lower compared to the MV Edmonston vaccine strain (Spielhofer, P. et al., Chimeric measles viruses with a foreign envelope, 72, 3 (1998)). Another research group, following a similar pseudotyping approach, discovered that an MV-VSV chimera was non-functional (Neault S. et al., "Robust envelope exchange platform for oncolytic measles virus," Journal of Virological Methods, 302, 114487, 2022). Clearly, the design and expression of a chimeric VSV-MV fusion protein that can compete with a natural VSV or MV virus in terms of infectivity and viral amplification remains a highly complex task.

Moreover, it is critical to acknowledge that any new and untested form of foreign biological material introduced into the body could potentially pose severe risks. Moreover, any alterations to the F-, H-, or G-proteins could potentially disrupt their three-dimensional structure, thereby affecting their ability to interact, which might impact cellular fusion activity. This makes the task of designing chimeras of these surface proteins an intricate and complex endeavour.

All the above strategies take advantage of the benefits of a Paramyxovirus-based viral delivery, such as self-replication and often established safety conditions, which offers a clear advantage over non-replicating virus-like particles. However, much like their natural counterparts, the reported MV-CDV chimeric viruses lead to extensive syncytia formation and ultimately the lysis of the infected cells. If this is projected into a therapeutic in vivo setting, it would cause significant tissue damage. While this is a clear advantage for oncolytic applications, it complicates milder and more controllable therapeutic interventions.

Therefore, the present invention aims to address these hurdles and safety issues, particularly cytotoxic syncytia, and to enable a wider application spectrum beyond cell lysis, enabling sophisticated cellular targeting and cargo delivery. This is achieved by a highly versatile and modular self-replicating delivery system that is able to target of a wide range of cellular surface receptors, which enables cell-specific targeting in a plethora of diseases besides cancer, a superior host immune-tolerance by using viral strains approved for vaccination and an increased protein expression by employment of segmented chimeric genomes.

### Summary of the Invention

The above-identified objects are solved by the technical teaching as provided with the present invention.

The hereby reported self-amplifying biologic (SelfAmp) is a modular cellular delivery system that can be specifically targeted to a wide range of cell types without interference with the host's immune system.

In a first aspect there is provided a self-amplifying biologic comprising or consisting of (i) at least one N-protein, at least one P-protein, at least one accessory protein C, at least one accessory protein V, at least one M-protein and at least one L-protein, and/or a sequence encoding the same, wherein the at least one protein originates from at least one paramyxovirus preferably from a measles virus; and (ii) at least one F-protein and/or at least one H-protein originating from the same paramyxovirus of (i), wherein the at least one F-protein and/or at least one H-protein each comprisean ectodomain, wherein said ectodomain is substituted against an ectodomainof at least one different paramyxovirus in comparison to the paramyxovirus of (i).

In one embodiment of the first aspect disclosed herein, there is provided a self-amplifying biologic, wherein the self-amplifying biologic further comprises at least one payload, and/or a sequence encoding the same.

In another embodiment of the first aspect and the embodiment disclosed therein, there is provided a self-amplifying biologic, wherein a target moiety is fused to the H-protein, preferably to the H-protein C-terminus or to a loop region of the H-protein, that binds to a target receptor associated with the host cell and thus targets the self-amplifying biologic to a receptor expressing cell, wherein the target moiety is a binding protein or a sequence encoding the same, comprising a natural ligand or an antibody or antibody fragment, comprising a scFv, or any peptide, comprising a DARPin or a nanobody, or any molecule, that interacts with a target receptor associated with the host cell.

Further, in one embodiment there is provided a self-amplifying biologic according to the first aspect or embodiments thereof, wherein the payload is encoded as an additional transcription unit and/or expressed under the control of at least one regulatory RNA element, preferably an IRES or a ribozyme, or a cell type-specific or cell state-specific RNA-based switch and/or further modified via a self-cleaving peptide, preferably a 2A-peptide, or a combination thereof.

In a second aspect, there is provided a vector, or more than one, preferably two vectors, wherein the one or more vector(s) comprise(s) at least one nucleic acid molecule encoding the self-amplifying biologic according to the first aspect or embodiments thereof, wherein said vector(s) comprise(s) a at least one nucleic acid molecule encoding the chimeric F-protein and H- protein as defined in the first aspect disclosed herein, and wherein said self-amplifying biologic self-replicates and self-assembles itself inside a biological host.

Further in one embodiment of the second aspect disclosed herein, there is provided at least one vector, characterized in that the at least one nucleic acid molecule, comprises a chimeric sequence of SEQ ID NOs: 1 and 2, or 12, or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any of the SEQ ID NOs: 1 and 2, or 12, respectively.

In another embodiment, there is provided at least one vector according the second aspect and the embodiment disclosed therein, characterized in that at least one nucleic acid molecule, as disclosed in the second aspect and previous embodiment, additionally comprises a sequence of SEQ ID NOs: 3, 4, 5 and 6 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any of the SEQ ID NOs: 3, 4, 5 and 6, respectively.

In a third aspect, there is provided a method of producing a self-amplifying biologic of the first and second aspect and the embodiments disclosed therein, the method comprising: (i) providing the at least one self-amplifying biologic as defined in the first aspect and the embodiments disclosed therein, and/or providing the at least one vector according to the second aspect and the embodiments disclosed therein, and introducing said at least one self-amplifying biologic and/or said at least one vector into at least one host cell, (ii) culturing the at least one host cell under conditions suitable for amplification of said at least one self-amplifying biologic and/or said at least one vector, (iii) allowing the assembly of at least one self-amplifying biologic from the at least one vector; and (iv) optionally: purifying said at least one self-amplifying biologic of step (iii); and (v) obtaining the at least one self-amplifying biologic.

In another embodiment, there is provided a method according to the third aspect, further comprising a step of (vi) introducing or contacting said at least one self-amplifying biologic as obtained in step (v) of the third aspect into at least one target cell or in vitro cellular system for transfecting said at least one target cell and/or for studying the functionality of the at least one self-amplifying biologic.

In a fourth aspect, there is provided a use of the at least one self-amplifying biologic according to any of the previous aspects and embodiments as a diagnostic tool, and/or for the introduction of at least one reporter molecule or a reporter system into a cell and/or for recombinant protein expression in cell culture, such as cytotoxic protein expression and/or production of at least one co-factor for virus production, and/or for circulating cancer cell diagnosis and/or for the generation of any modified cell, preferably a CAR-T or a CAR-NK cell, and/or for ex vivo gene therapy and/or ex vivo gene transfer.

In a fifth aspect, there is provided a pharmaceutical composition comprising the at least one self-amplifying biologic according to the first aspect and embodiments thereof.

In another aspect, there is provided a pharmaceutical composition comprising the at least one self-amplifying biologic obtained by or obtainable by a method according to the third aspect and the first embodiment thereof, and optionally, at least one pharmaceutically acceptable carrier or excipient.

In one embodiment, there is provided a pharmaceutical composition according to the previous aspect for use in a method of treating or preventing a disease.

In another embodiment, there is provided a pharmaceutical composition according to the previous aspect for use in a method for the treatment and/or prevention of an inflammatory disease, preferably an autoimmune disease, or a cancer.

In a further embodiment, there is provided a pharmaceutical composition according to the previous embodiment, wherein the inflammatory disease is selected from the group consisting of Multiple Sclerosis, Rheumatoid Arthritis, Gout, Psoriasis, Asthma, Crohn's Disease, Chronic Obstructive Pulmonary Disease, Allergies, Hepatitis, Dermatitis or Sarcoidosis.

In a further embodiment, there is provided a kit comprising the self-amplifying biologic according to the first aspect and the embodiments disclosed therein, and/or a vector according to the second aspect and the embodiments disclosed therein, optionally wherein the kit comprises further reagents, including at least one buffer or solution.

Further aspects and embodiments of the present invention can be derived from the subsequent detailed description, the Figures, the Sequence Listing, as well as the attached set of claims.

### Brief Description of Figures

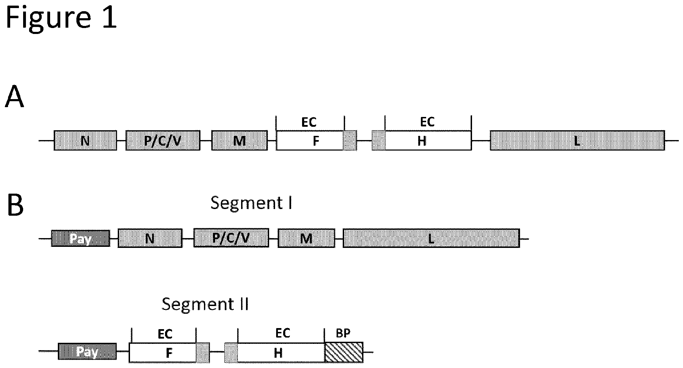

Figure 1: Schematic representation of a Self Amp
   A) Topological scheme of a SelfAmp. Genes from two different paramyxoviruses are represented by grey and white boxes, respectively. EC: ectodomain
   B) Topological scheme of a SelfAmp comprising two payloads distributed on two segments. Genes from two different paramyxoviruses are represented by grey and white boxes, respectively. The shaded box shows an optional binding protein fused C-terminally to the H-protein. EC: ectodomain, BP: binding protein, Pay: payload.

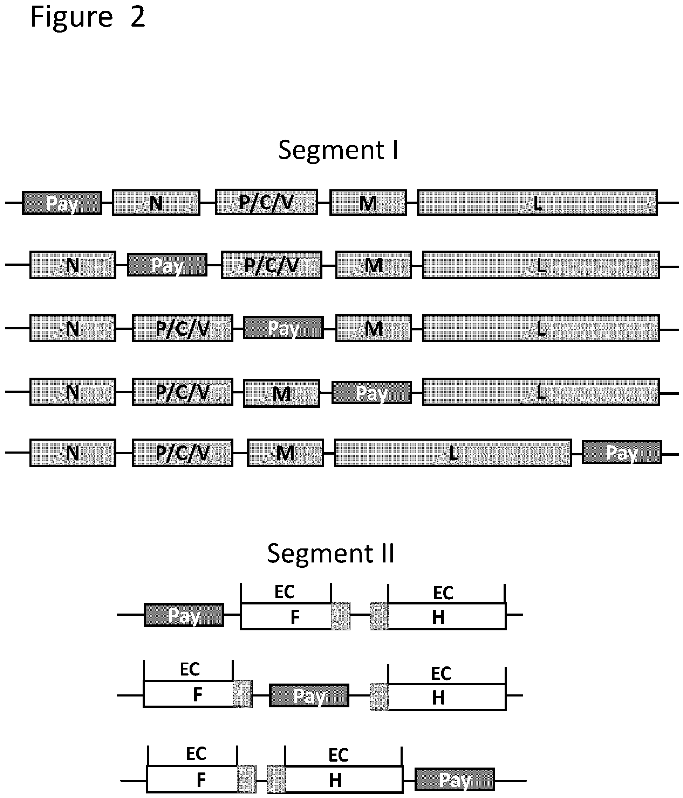

Figure 2: Schematic representation of payload arrangements
   Topological scheme of a construct distributed on two segment, demonstrating a possible arrangement of payload sequences.EC: ectodomain, Pay: payload.
Figure 3: Vector map of a Self Amp (LLB-401)
   The map shows genes originating from MV and CDV as a DNA vector, comprising GFP as a payload. The respective DNA sequences are listed in the sequence protocol. Restriction sites used for cloning and placeholders for ATU insertion are indicated. AmpR: ampicillin resistance gene, ATU: Additional Transcription Unit, cDNA: complementary DNA, CMV promoter: cytomegalovirus promoter, GFP: green fluorescent protein.

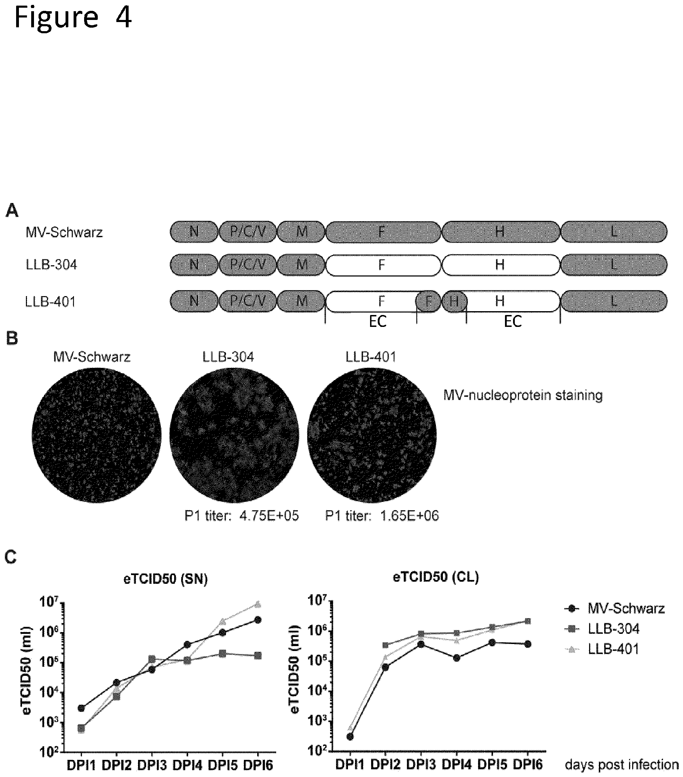

Figure 4: Generation and comparative characterization of LLB-401
   A) Schematic representation of MV-Schwarz, LLB-304 and LLB-401 genomes. LLB-304 contains full length F and H from CDV (Onderstepoort strain), LLB-401 contains the extracellular domains of CDV F and H, while transmembrane and cytosolic parts of the genes were derived from MV-Schwarz. B) Left: Vero cells were infected with recombinant rescued virus and incubated for three days to allow syncytia formation, and stained for nucleoprotein. Right: Quantitative analysis of syncytia formation (in µm*²*) was performed by gating on nucleoprotein positive areas using an automated microscope (ImageXpress^{®} Pico Automated Cell Imaging System). Small- (MV-Schwarz; LLB-401) and large-forming syncytia (LLB-304) as well as passage #1 titers are depicted. C) Growth characteristics were evaluated by infecting Vero 10-87 cells with rescued virus at an MOI of 0.01 and supernatant and cell lysates were harvested for 6 subsequent days (DPI1-DPI6). TCID50=tissue culture infectious dose at which 50% of cells are infected. CL=cell lysate. SN=supernatant.

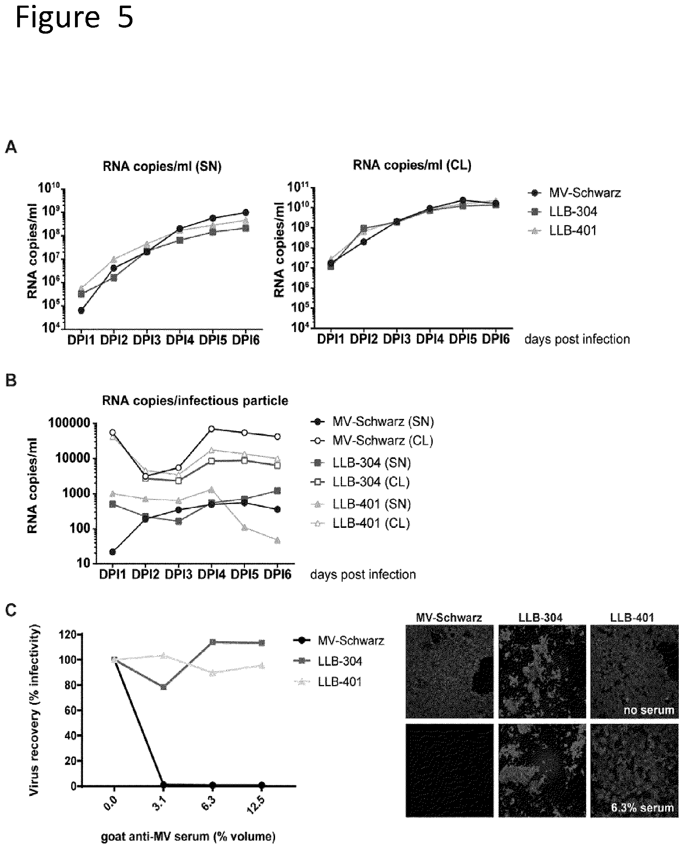

Figure 5: Neutralization efficiency by MV-Schwarz serum
   A) B) Growth characteristics were evaluated by infecting Vero 10-87 cells with rescued virus (MV-Schwarz, LLB-304 and LLB-401) at an MOI of 0.01 and supernatant and cell lysates were harvested for 6 days (DPI1-DPI6). RNA copies/ml as well as RNA copies/infectious particle was evaluated. CL=cell lysate. SN=supernatant. C) MV-CDV (F/H) is fully protected from anti-MV neutralizing antibodies. Virus was pre-incubated with goat-derived anti-MV serum and then added to Vero 10-87 cells. Infection efficiency was evaluated by staining for nucleoprotein (NP) and cell nucleus (DAPI). MV-Schwarz was efficiently neutralized by serum, while not affecting MV-CDV (F/H) even at very high concentrations.

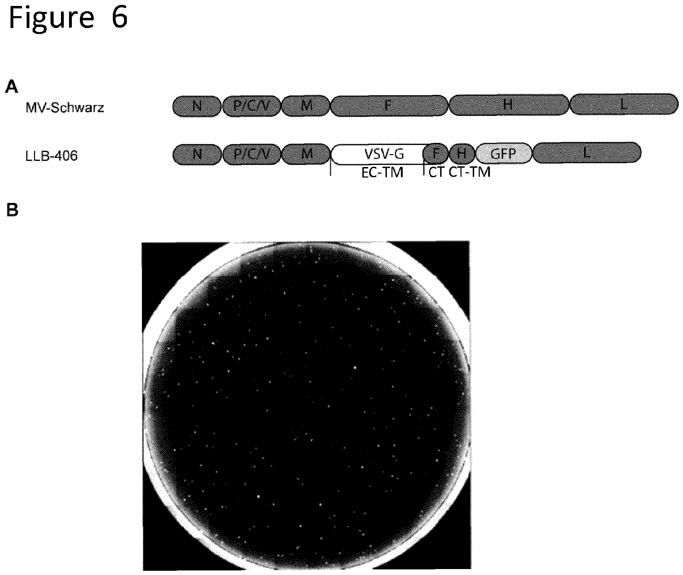

Figure 6: Payload expression of a Self Amp with VSV G-protein ectodomain-transmembrane domains
   A) Topological scheme of a SelfAmp with a vesicular stomatitis virus (VSV) chimeric G-protein. Genes from two different constructs are represented by grey and white boxes, respectively. LLB-406 contains extracellular and transmembrane domains of vesicular stomatitis virus glycoprotein, while the intracellular domain is part of the fusion gene of MV-Schwarz. Hemagglutinin of MV-Schwarz is only present with the intracellular and transmembrane domain fused to 3 amino acids of the extracellular part. EC: Ectodomain, TM: transmembrane domain; CT: cytoplasmic tail,
   B) Vero-E6 cells were infected with cell lysates of recombinant rescued LLB-406 virus and incubated for 6 days. GFP expression is depicted and visualized via an automated microscope.

### Brief Description of Sequences

| SEQ ID NO: | Description |
|---|---|
| 1 | CDV F-protein::MV F-protein ORF |
| 2 | MV H-protein::CDV H-protein ORF |
| 3 | MV N-protein ORF |
| 4 | MV P/C/V-protein ORF |
| 5 | MV M-protein ORF |
| 6 | MV L-protein ORF |
| 7 | CMV promoter |
| 8 | GS of ATU |
| 9 | GE of ATU |
| 10 | MV Leader sequence |
| 11 | MV Trailer sequence |
| 12 | VSV G-protein::MV F-protein ORF |

### Definitions

Unless defined otherwise, technical and scientific terms used herein have the same meaning as commonly understood by one of ordinary skill in the art to which this invention belongs.

An additional transcription unit (ATU) is defined as a separate section of the genome of a virus or virus-like particle. An ATU may contain one or more genes, as well as regulatory elements that control gene transcription and expression.

An antibody, also known as an immunoglobulin, is a Y-shaped protein mostly made by plasma cells, a type of white blood cell. Antibodies are part of the body's immune system. They recognize and bind to specific foreign substances (antigens), such as bacteria, viruses, or toxins, helping to neutralize or eliminate them. There are five main types of antibodies: IgM, IgG, IgA, IgD, and IgE, each with a distinct function in the immune response.

A biological host comprises every biological entity from a single cell to tissues and organisms wherein foreign material, e.g., a vector or a SelfAmp, can be introduced.

Chimeric Antigen Receptor Natural Killer (CAR-NK) cells and Chimeric Antigen Receptor T (CAR-T) cells are types of immune cells that have been genetically engineered to express a receptor that can specifically recognize and bind to a target antigen, often on the surface of cancer cells.

CAR-NK cell therapy utilizes natural killer (NK) cells, which are a type of white blood cell with inherent cancer-killing capabilities. These NK cells are modified in the laboratory to express a CAR that is specific to the patient's cancer. Derived from various sources, including cord blood, peripheral blood, or induced pluripotent stem cells (iPSCs), CAR-NK cells have the potential to be used as an "off-the-shelf' therapy as they do not cause graft-versus-host disease.

Similarly, CAR-T cell therapy involves collecting a patient's own T cells, another type of white blood cell, and genetically engineering them in the laboratory to express a CAR specific to the cancer. Once reintroduced into the patient, these CAR-T cells can identify and destroy the cancer cells. CAR-T cell therapies have been approved for certain types of leukemia and lymphoma.

Cellular delivery is defined as the process of introducing bioactive substances, such as drugs or genetic material, into cells for therapeutic or research purposes. Methods can be non-viral, including physical techniques like electroporation or chemical ones like lipofection, or viral, using modified viruses as delivery vectors. The objective is to deliver the substance without causing harm and to ensure it reaches its intended cellular site of action.

Circulating cancer cell diagnosis, often referred to as detection of circulating tumor cells (CTCs), is a diagnostic approach in oncology that involves identifying cancer cells that have detached from a primary tumor and entered the bloodstream. CTCs are often detected through a blood draw, making it a less invasive method for cancer detection compared to tissue biopsies. Technologies used to detect CTCs often employ a combination of size-based, density-based, and immunoaffinity-based methods, often relying on the expression of specific markers such as EpCAM and cytokeratins to distinguish CTCs from normal blood cells. Detection of CTCs can provide valuable insights into the biology of the cancer and can also be used to monitor disease progression and response to treatment in real-time, making CTCs a potential tool for 'liquid biopsy' in cancer therapy.

Cytotoxic protein expression is a process in which cells synthesize proteins that have the ability to cause damage to or kill other cells. These cytotoxic proteins can be part of a cell's normal function, as seen in immune cells like cytotoxic T cells, or they can be expressed as a result of pathological conditions, such as viral infections or certain genetic diseases.

A Designed Ankyrin Repeat Protein (DARPin) is non-immunoglobulin, synthetic binding protein. It is engineered to bind to specific target proteins with high affinity and specificity, based on the naturally occurring ankyrin repeat protein motif. Ankyrin repeats are among the most common structural motifs in known proteins and are typically involved in protein-protein interactions. Each repeat consists of a loop-helix-loop-helix structure, and in a DARPin, multiple repeats are stacked together to create a large, continuous surface for potential protein-protein interaction. One advantage of a DARPin over an antibody for certain applications is its small size and high stability, allowing it to access and bind to areas of target molecules that remain inaccessible for an antibody. Another advantage is that it can be engineered in the laboratory using standard protein production techniques, making them relatively easy and cost-effective to produce.

The ectodomain in viruses is defined as the portion of a viral envelope protein that extends outside the virus particle or the host cell membrane. The ectodomain may be divided into two parts: the globular head domain - the outermost part - and the stalk domain.

Ex vivo gene therapy is a type of genetic treatment where cells are removed from a patient, genetically modified in a laboratory setting, typically using viral or non-viral vectors to insert a therapeutic gene, and then returned to the patient with the intent to cure or alleviate disease symptoms.

Ex vivo gene transfer refers specifically to the process of introducing new genetic material into the patient's cells while they are outside the body, in a controlled laboratory environment. This transfer is usually achieved through the use of a vector, which carries the desired gene into the cells. Once the cells have been modified, they are infused back into the patient.

The globular head domain of the F- and H-protein of paramyxoviruses is a distinct structural domain and form the outermost part of the ectodomain. These domains are often described as "globular" because they adopt a compact, roughly spherical three-dimensional structure. For the F protein, the globular head is often considered to be part of the F2 subunit, which is produced by cleavage of the inactive F0 precursor. The F1 subunit contains the fusion peptide and is anchored in the viral membrane. For the H protein, the globular head contains the receptor binding site and is located at the end of a stalk that projects from the viral surface. The stalk allows the globular head to be distanced from the viral membrane, providing the necessary reach to interact with the host cell receptor. The globular head's interaction with the host cell receptor is critical for viral attachment.

An Internal Ribosome Entry Site (IRES) is a specialized sequence within a messenger RNA (mRNA) that allows for cap-independent initiation of protein translation. This sequence forms a unique RNA secondary structure that enables the direct binding of the ribosome, bypassing the traditional 5' cap scanning mechanism. IRES elements, initially discovered in viruses, are found to play critical roles in both normal cellular processes and viral replication, particularly under conditions when conventional translation initiation is impaired.

An in vitro cellular system is defined as a controlled experimental setup where cells are studied outside their natural biological context, usually in a petri dish or test tube. This can include single cells or cell cultures, and provides a platform to examine cell behaviour, drug responses, genetic function, and more, under defined conditions.

A kit is a packaged set of reagents, materials, and optionally instructions, designed to carry out a specific experimental or diagnostic procedure. These kits aim to streamline and simplify the process, ensuring reliability and reproducibility of results.

A nanobody is a small, single-domain antibody fragment derived from heavy chain-only antibodies found in camelids, such as camels, llamas, and alpacas. Unlike conventional antibodies, which are composed of two heavy and two light chains, these heavy chain-only antibodies lack light chains. The variable region of these heavy chains (V_{H}H) is fully capable of antigen binding and is what we refer to as a nanobody. Nanobodies offer several advantages over conventional antibodies. They are much smaller (about a tenth the size), making them more soluble and stable, and allowing them to access and bind to antigenic sites that may be inaccessible to larger antibodies.

A paramyxovirus is a virus belonging to the family of *Paramyxoviridae* in the order *Mononegavirales* of negative-sense single-stranded RNA viruses. The Paramyxovirus family comprises 78 virus species, including e.g., measles virus (MV), Canine Morbillivirus (CDV) and Newcastle Disease Virus (NDV).

A payload is defined as a functional molecule, preferably a nucleic acid molecule or protein molecule that is transported as a cargo to a target cell, where it carries out or influences a biological activity.

A pharmaceutical composition refers to a formulation or mixture prepared for therapeutic or prophylactic use in human or veterinary medicine. It includes an active pharmaceutical ingredient, which provides the intended therapeutic effect, and excipients, which are inactive ingredients that serve roles such as enhancing solubility, improving stability, or aiding in drug delivery.

Recombinant protein expression is a biotechnological process where a specific protein is produced in a host organism that has been genetically modified with a nucleic acid sequence coding for that protein. This technique is used in research, pharmaceutical production, and industrial applications to produce large quantities of specific proteins.

A regulatory RNA element is defined as a class of RNA molecules that play a role in gene expression regulation. These RNA molecules do not code for proteins, but instead, regulate the expression of genes by interacting with specific target sequences in DNA or other RNA molecules or influence gene expression e.g., microRNAs (miRNAs), small interfering RNAs (siRNAs), and long non-coding RNAs (IncRNAs). Some regulatory RNA elements can also influence gene expression by direct interaction with the translation machinery, as is the case for an IRES or a ribozyme.

A reporter system is a tool used in molecular biology that couples a gene of interest with a reporter gene. The reporter gene is selected for its easily identifiable and measurable characteristics, often producing a specific protein product, such as a fluorescent protein or an enzyme. Changes in the activity or localization of the gene of interest are then indirectly measured via changes in the reporter gene's activity or expression, providing a visible or quantifiable readout.

A ribozyme is a type of RNA molecule that can catalyze specific biochemical reactions, similar to the action of protein enzymes. The term "ribozyme" combines "ribonucleic acid" and "enzyme", reflecting its function as a catalyst. Ribozymes can carry out a range of reactions, with the most common being the cleavage and ligation of RNA strands. To example, the hammerhead ribozyme is a small, self-cleaving RNA structure found in a number of RNA plant pathogens and used for recombinant viral replication.

Self-replication is a property of biological systems that allows the construction of an identical or similar copy of itself.

A self-amplifying biologic (Self-Amp) is a biologically-active particle capable of self-replication by harnessing biomolecular machinery such as that found inside a biological host. In contrast to a naturally occurring virus, a Self-Amp is a synthetic virus-like particle, engineered for desired biological activity. Further, a Self-Amp can yield additional functionality by binding additional molecules from its biological host.

A self-cleaving peptide is a short sequence of amino acids that can catalyze its own cleavage, causing the peptide chain to break at a specific location without the need for an enzyme. 2A peptides are well-known examples of self-cleaving peptides, and were first discovered in foot-and-mouth disease virus. This peptide, when incorporated into a larger protein sequence, can 'cleave' itself, thereby allowing multiple distinct proteins to be produced from a single gene sequence.

A single-chain variable fragment (scFv) is a type of engineered antibody fragment that consists of the variable regions of the heavy (VH) and light chains (VL) of immunoglobulins, connected by a short linker peptide. The linker peptide is flexible and long enough to allow the VH and VL domains to associate and form a binding site that is very similar to that of a full-size antibody. Because they maintain the original antigen specificity and binding affinity of the parent antibody, scFvs are valuable in research, diagnostics, and therapeutics. The smaller size of the scFv compared to a full-length antibody can enhance tissue penetration and accelerate blood clearance. On the other hand, their smaller size can also result in reduced binding avidity due to the loss of the bivalent binding capability of full-size antibodies. To overcome this limitation, scFvs can be engineered into multivalent or multispecific formats.

A stalk domain is an essential part of the Paramyxovirus F- and H-protein, specifically, a part of its ectodomain, forming a long, slender structure that connects the globular head to the transmembrane domain. For the H-protein, the stalk facilitates the attachment of the virus to the host cell by positioning the globular head for interaction with the host cell receptor. For the F-protein, the stalk region is important for the protein's fusion activity. The stalk domain is characterized by its elongated, coiled-coil structure, which allows the stalk to maintain its shape and rigidity, positioning the globular heads for optimal function. The globular head-stalk architecture allows the virus to maintain a distance from the host membrane until signals for fusion are present.

A target moiety is defined as a part of a protein or peptide that covalently binds to a target molecule such as a receptor molecule. The target moiety typically is an antibody, an antibody fragment, a nanobody, a DARPin or an affimer, but can be any molecule or molecular scaffold that can bind to a target molecule.

A target receptor is defined as a biological molecule that can be bound by a molecule. The binding event triggers a biological signal that influences a cellular pathway or a cellular signalling cascade.

Whenever the present disclosure relates to the percentage of identity of nucleic acid or amino acid sequences to each other these values define those values as obtained by using the EMBOSS Water Pairwise Sequence Alignments (nucleotide) programme (www.ebi.ac.uk/Tools/psa/emboss_water/) nucleic acids or the EM-BOSS Water Pairwise Sequence Alignments (protein) programme (www.ebi.ac.uk/Tools/psa/emboss_watern for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and SMITH, T.F. 5 & WATERMAN, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a protein can be "codon-optimized" if the respective sequence is to be used in another organism in comparison to the original organism a molecule originates from.

### Detailed Description

As specified in the background section, the deployment of Paramyxoviruses for viral therapy remains an overall challenging task. Further, approaches for immune-evasive viruses by designing surface protein chimeras remain a difficult task due to the intricacies of the virus-specific surface protein structure. Another significant problem for milder therapeutic interventions remains the extensive syncytia formation upon paramyxovirus infection, which could provoke inflammation and unintentional damage to healthy tissue.

The present invention offers a solution to the problems of the above mentioned delivery systems: A Self-Amplifying biologic (SelfAmp) is designed to be delivered precisely to any cell within a mammalian body, allowing the targeted cells to produce effectors that are encoded within the SelfAmp. The tailored design of an immune-evasive outer shell enables compatibility with a wide range of host organisms and targeted cellular delivery via receptor-binding target moieties. The SelfAmp can also carry therapeutically active molecules, or "payloads," to aid in the treatment of various conditions, such as immunomodulatory molecules, which can help regulate or modify immune responses. Optionally, the SelfAmp or any components thereof, can be chemically modified to improve its performance or meet certain needs and/or may partially or fully consist of synthetic molecules. A major advantage is thus the inherent modular flexibility of a SelfAmp as disclosed and claimed herein.

In addition, a SelfAmp can have components that are inducible, or capable of being activated when inserted in a host organism. This feature allows for the external control of in vivo expression and replication, which can be beneficial for precise management of the payload's activity within the body.

To address the needs to provide a targeted and modular immune-evasive cellular delivery system, the present invention thus provides in a first aspect a self-amplifying biologic comprising or consisting of (i) at least one N-protein, at least one P-protein, at least one accessory protein C, at least one accessory protein V, at least one M-protein and at least one L-protein, and/or a sequence encoding the same, wherein the at least one protein originates from at least one paramyxovirus preferably from a measles virus; and (ii) at least one F-protein and/or at least one H-protein originating from the same paramyxovirus of (i), wherein the at least one F-protein and/or at least one H-protein each comprise an ectodomain, wherein said ectodomain is substituted against an ectodomain of at least one different paramyxovirus in comparison to the paramyxovirus of (i).

Additionally, in one embodiment of the first aspect, the cell receptor binding and fusion activity of the self-amplifying biologic is conveyed via a single protein, the spike protein of a SARS-CoV virus, a flavivirus E-protein or a rhabdovirus G-protein, such as a vesicular stomatitis virus G-protein, but not limited thereto. Any single fusion protein providing both the binding and the fusion activity as described for the constructs of the first aspect of the present invention may be suitable.

In certain embodiments, the use of a single fusion protein may be advantageous, in case that the single fusion protein comprises both binding and fusion functions. In certain embodiments, the ectodomain and transmembrane domain of the rhabdovirus G-protein is fused to the cytoplasmic tail of a paramyxovirus F-protein, which may be advantageous for maintaining the structural and functional integrity of a paramyxovirus-based SelfAmp. In another embodiment of the first aspect, a single fusion protein may be used that is or that originates from human syncytin. Other single fusion proteins may be used in case these provide both the binding and the fusion activity as described for the constructs of the first aspect of the present invention.

Generally, in one embodiment according to the first aspect, the at least one G-protein may originate from a virus other than a paramyxovirus, and at least one F-protein and at least one H-protein may both originate from a paramyxovirus, each comprising an ectodomain, a transmembrane domain and a cytoplasmic tail, whereas the ectodomain and the transmembrane domain of the at least one G-protein are fused to the cytoplasmic tail of the at least one F-protein, and the at least one H-protein lacks the ectodomain".

In one embodiment, the self-amplifying biologic comprises at least one nucleic molecule, preferably wherein the at least one nucleic acid molecule is selected from naturally occurring or synthetic DNA and/or RNA, such as xeno-nucleic acids, comprising a synthetic backbone, including phosphorothioate modifications, and/or nucleotide bases coding for non-natural amino acids, preferably wherein the at least one nucleic acid molecule encodes a synthetic virus or a virus-like particle.

In certain embodiments, the nucleic acid sequence encoding a protein comprises an upstream regulatory element, such as a GS sequence, an open reading frame (ORF) and a downstream regulatory element, such as a GE sequence.

In one embodiment, the self-amplifying biologic is a complex of protein and nucleic molecules, wherein the nucleic acid molecules are packaged by proteins and protected by a protein shell, preferably wherein the self-amplifying biologic is a synthetic virus or a virus-like particle.

Further, in one embodiment said nucleic acid molecule is a single-stranded negative-sense RNA.

Further, the self-amplifying biologic comprises or consists of proteins from at least one Paramyxovirus, selected from the Paramyxoviridae family of viruses, including human parainfluenza viruses, mumps virus, Morbilliviruses, preferably, measles virus, canine morbillivirus, phocine distemper virus; henipaviruses, such as Hendra virus and Nipah virus; or Newcastle disease virus, but not limited thereto.

Paramyxoviruses are enveloped viruses, distinguished by their single-stranded, negative-sense RNA genome. This group of viruses, part of the Paramyxoviridae family, contains notable human and animal pathogens such as the measles virus, mumps virus, and parainfluenza viruses, among others. Genetically, paramyxoviruses have a non-segmented, negative-sense RNA genome. This means that their genetic material is a single strand of RNA that carries the complementary negative-sense sequence of the viral genes. The viral RNA encodes various proteins crucial for replication, assembly, and pathogenesis.

The Paramyxovirus life cycle is characterized by a series of steps. First, the attachment protein on the virus surface binds to specific receptors on the host cell, enabling attachment. Then, the F-protein undergoes a conformational change, allowing the viral envelope to fuse with the host cell membrane. This fusion releases the viral nucleocapsid into the host cell cytoplasm.

Once inside the host cell, the viral RNA is released, and viral proteins and enzymes replicate the genome and transcribe viral mRNA molecules. The mRNA is then translated by the host cell machinery, producing viral proteins including structural components and enzymes. These components are assembled within the host cell, forming mature virions. The mature virions gain their envelopes embedded with viral glycoproteins by budding through the host cell's outer membrane. These new virions can infect other cells, triggering new infection cycles (CHANG, A and DUTCH, RE, Paramyxovirus fusion and entry: multiple paths to a common end, Viruses, 4, 613-636 (2012)).

Notably, paramyxovirus fusion proteins can induce fusion with plasma membranes of neighbouring cells, leading to the formation of syncytia, and thus, a single virion can potentially infect and kill many cells, which is one reason for the favourable use of paramyxovirues in oncolytic virotherapy (MATVEEVA, OV, Oncolysis by paramyxoviruses: multiple mechanisms contribute to therapeutic efficiency, Molecular Therapy - Oncolytics, 2, 15011 (2015)).

However, while the tissue-damaging behaviour of viruses, including the expansive syncytia-formation by paramyxoviruses, is a desired property when directed against cancer tissue, it poses a severe safety concern for every other application. There exists the risk of adverse side effects or illness in patients due to potential unintended immune responses triggered by the viral presence.

Therefore, under the provision that the complex genetics and characteristics of paramyxoviruses are duly taken into consideration to guarantee a safe use of the virus, it may be a suitable biotechnological tool for cellular targeting.

In a preferred embodiment, said N-protein is encapsulated by the nucleic acid molecule disclosed herein, yielding a protective helical structure and providing stability to said nucleic acid molecule, wherein the protective helical structure is critical for the replication of said nucleic acid molecule.

In another embodiment, the L-protein is a viral replicase complex, such as an RNA polymerase complex, to achieve self-replication of the self-amplifying biologic.

In addition, in one embodiment the H-protein that serves as the initial point of interaction between the self-amplifying biologic and a host cell. Following the attachment process mediated by the H-Protein, the F-Protein becomes active. Said F-protein is responsible for fusing the envelope of the self-amplifying biologic with the host cell membrane, wherein said fusion process releases the self-amplifying biologic into the host cell cytoplasm, resulting in the formation and self-replication of the self-amplifying biologic.

Both the F- and H-proteins consist of meticulously delineated domains, each playing a significant role in viral function. At the apex, the globular head domain, which interacts with the target receptor, forms the interaction interface with the host cell in the H protein. Conversely, in the F protein, it houses the fusion peptide, responsible for envelope-cell membrane fusion. Descendant to the globular head domain, the stalk domain connects the head to the transmembrane domain. It spatially orients the globular head for receptor engagement in the H protein and incites conformational changes critical for membrane fusion in the F protein. Further, the transmembrane domain, nested within the viral envelope's lipid bilayer, fastens the F and H proteins to the virus. It also participates in the fusion-triggering transformations within the F protein. Towards the virus's interior, the cytoplasmic tail contributes to viral particle assembly and budding. In the F protein, it potentially modulates the membrane fusion initiation. The skilled person is thus aware of the function of a globular head domain in its natural context and would not change the overall architecture of this domain to not risk a loss of function.

In one embodiment, the self-amplifying biologic gains additional functionality by molecules provided by the biological host, wherein these molecules comprise an amino acid molecule, such as a peptide and/or a protein, preferably a transcription factor, and/or a carbohydrate molecule such as a sugar, and/or a lipid.

In another embodiment, said self-amplifying biologic gains additional functionality by post-translational modifications such as glycosylation, phosphorylation, acylation, acetylation, methylation, ubiquitination, sumoylation, prenylation, nitrosylation, sulfation, disulfide bridges, but not limited thereto.

In another embodiment, said self-amplifying biologic gains additional functionality by chemical modifications, comprising, but not restricted to, chemical modifications of canonical amino acids, such as modifications of lysines, the protein N-terminal primary amine, cysteine residues, aromatic residues, or the incorporation of non-canonical amino acids via e.g., genetic code expansion, site-specific amino acid incorporation or residue-specific amino acid incorporation, or ligand-directed modifications, or nucleic acid modifications, such as pseudouridylation, or chemical modifications of the nucleic acid sugar moiety, phosphate backbone, or nucleobase.

In certain embodiments, the surface of said self-amplifying biologic is chemically modified via bioconjugation, comprising covalent attachment of chemical moieties such as PEGylation or antibody conjugation, or glycan modification, but not limited thereto.

In one embodiment of the first aspect disclosed herein, there is provided a self-amplifying biologic, wherein the self-amplifying biologic further comprises at least one payload, and/or a sequence encoding the same.

In certain embodiments of the various aspects of the present invention, more than one payloads are distributed on different vectors.

In a preferred embodiment, the payload is selected from, but not limited to, the group of cytokines, such as IL-2, IL-10, TNF-alpha and interferons, chemokines, such as CCL2, antibodies, preferably therapeutic antibodies, such as pembrolizumab or rituximab, antibody fragments, preferably an scFv or a Fab, nanobodies, DARPins, immunomodulators, comprising a viral protein, such as NS-1, or a PRR/TLR stimulator, ligands, comprising a natural or a modified ligand, receptors, comprising natural or modified receptors, transporters, such as CFTR or NIS, comprising natural and modified transporters, or chimeric proteins, such as a chimeric antigen receptor, enzymes, comprising a prodrug converting enzyme, such as HSV-TK or a cytosine deaminase.

In another preferred embodiment, the payload is selected from, but not limited to, the group of reporter genes, such as a fluorescent protein, a luciferase system, a PET-reporter, an MRI-reporter or an MSOT-reporter, or biomarkers, such as glucuronidase or FSH.

In another preferred embodiment, the payload is selected from, but not limited to, the group of toxins, growth factors, signaling molecules, co-stimulatory proteins, such as CD80 or CD86, apoptosis inducing proteins, antigens, hormones, such as insulin, proteases, genome modifying proteins, such as CRISPR/Cas or a TALEN, Tags, such as a Snap-Tag, a CLIP-Tag, a Halo-Tag or a Strep-Tag, or RNA molecules, such as an mRNA, a gRNA, an siRNA or an miRNA.

In another embodiment of the first aspect and the embodiments disclosed therein, there is provided a self-amplifying biologic, wherein a target moiety is fused to the H-protein, preferably to the H-protein C-terminus or to any loop region of the H-protein, that binds to a target receptor associated with the host cell and thus targets the self-amplifying biologic to a receptor expressing cell, wherein the target moiety is a binding protein or a sequence encoding the same, comprising a natural ligand or an antibody or antibody fragment, comprising a scFv, or any peptide, comprising a DARPin or a nanobody, or any molecule that interacts with a target receptor associated with the host cell.

Targeting moieties, including single-chain variable fragments (scFvs), nanobodies, or DARPins, have important roles in the context of virus-like particles (VLPs), recombinant viruses or other biological delivery vehicles for targeted delivery purposes and thus can be used analogously for SelfAmps. These targeting moieties are engineered to bind specifically to certain molecules or receptors on the surface of target cells. When incorporated into biological delivery vehicles, they enhance the selectivity and efficiency of cellular targeting. In the case of recombinant viruses, targeting moieties can be displayed on the viral surface. This allows for the specific recognition and binding to receptors on target cells. By conjugating targeting moieties to recombinant viruses, researchers can achieve precise delivery of cargo, such as therapeutic agents or vaccines, to desired cell types or tissues.

Similarly, targeting moieties can be incorporated into other biological delivery vehicles like liposomes or exosomes, which can encapsulate and protect therapeutic molecules or imaging agents while being functionalized with targeting moieties on their surface. This combination facilitates the specific interaction with target cells, leading to enhanced internalization and payload delivery. Target moieties, such as antibody-derived scFvs or nanobodies, or DARPins, can be engineered to recognize various targets, including cell surface receptors, disease-specific markers, or tumor-associated antigens. Their use in biological delivery vehicles provides advantages such as increased targeting precision, reduced off-target effects, and improved therapeutic efficacy. Moreover, the modular nature of these targeting moieties allows for easy customization and adaptation to different delivery systems. They can be fused or conjugated to biological vehicles without significantly compromising their binding affinity or stability (PLÜCKTHUN, A, Designed ankyrin repeat proteins (DARPins): binding proteins for research, diagnostics, and therapy, Annu. Rev. Pharmacol. Toxicol, 55, 489-511 (2015)). For example, an scFv fused to the MV H-protein directed against human carcinoembryonic antigen (CEA) (MIEST, TS et al., Envelope-chimeric entry-targeted measles virus escapes neutralization and achieves oncolysis, Molecular Therapy 19, 10, 1813-1820 (2011)) or an scFV fused to the CDV H-protein directed against EGFR or CD38 (BAH, ES et al., Retargeted and stealth-modified oncolytic measles viruses for systemic cancer therapy in measles immune patients, Mol. Cancer Ther., 19, 2057-2067 (2020) were used as specific target moieties for directed cell delivery of recombinant viruses.

In a preferred embodiment, said binding protein binds non-covalently or covalently, e.g. via a cysteine-bridge, to a target receptor.

In another embodiment, said target receptor comprises a G-protein coupled receptor, such as an adrenergic receptor, a histamine receptors, a dopamine receptor, a serotonin receptor, an opioid receptor, an angiotensin receptor, an acetylcholine receptor, a chemokine receptor, a cannabinoid receptor, or a glucagon receptor, a receptor tyrosine kinase, such as an Epidermal Growth Factor Receptor (EGFR), a Human Epidermal Growth Factor Receptor (HER2), a Vascular Endothelial Growth Factor Receptor (VEGFR), a Platelet-Derived Growth Factor Receptor (PDGFR), an Insulin Receptor (IR), an Insulin-like Growth Factor 1 Receptor (IGF-1R), an Anaplastic Lymphoma Kinase (ALK), an integrin, such as Integrin αIIbβ3, Integrin αvβ3, Integrin α4β1 (VLA-4), Integrin α4β7 or Integrin αLβ2 (LFA-1), a pattern recognition receptor (PRR), such as a Toll-like receptor (TLR), a nucleotide-binding oligomerization domain (NOD)-like receptor or a Rig-I-like receptor (RLR), a membrane-bound cytokine receptor, preferably an immunoglobulin superfamily (IgSF) receptor, such as an IgSF cell-adhesion molecule (IgCAM), a B-cell receptor (BCR) or a T-cell receptor (TCR), a cadherin, an integrin, or an artificial and/or recombinant pseudo-receptor, but not limited thereto.

Further, in one embodiment there is provided a self-amplifying biologic according to the first aspect or embodiments thereof, wherein the payload is encoded as an additional transcription unit and/or expressed under the control of at least one regulatory RNA element, preferably an IRES or a ribozyme, or a cell type-specific or cell state-specific RNA-based switch and/or further modified via a self-cleaving peptide, preferably a 2A-peptide, or a combination thereof.

Paramyxovirus transcription units (TUs) or genes contain untranslated regions (UTRs) at their 3' and 5' ends, consisting of non-coding sequences (NCS) and conserved gene end (GE) and gene start signals (GS). These regions are relatively conserved across both wildtype and vaccine strains of the virus. Each TU's GS signal is made up of a relatively conserved sequence, and the 5' UTR of the MV N-protein mRNA has been found to contain regulatory motifs that affect MV transcription and replication. MV, like other paramyxoviruses, exhibits a transcriptional gradient, meaning transcription levels of each viral gene decrease gradually along the viral genome from the 3' to 5' end. This is due to the viral RNA polymerase partially dissociating at each GE sequence, reducing its ability to initiate transcription at the next downstream gene. The mRNA for each MV gene is synthesized by the viral RNA-dependent RNA polymerase (vRdRp), which initiates synthesis at the GS +1 site. The resulting mRNA is composed of the protein-coding open reading frame (ORF), the GS sequence, and additional sequences at the 3' and 5' ends. Recombinant MVs can stably express large foreign protein-coding sequences (up to 6 kb), which are inserted into additional transcription units (ATUs) in the MV genome. Each ATU is designed to include a stop-start signal sequence pattern (GS-ORF-GE) to facilitate the expression of these additional genes. To regulate the expression levels of these foreign proteins, the position of the ATU in the genome can be adjusted using the transcriptional gradient mechanism (WEI, J, et al., Introduction of a conserved sequence into the 5'UTR of additional transcription down-regulation expression of foreign proteins of measles virus minigenome, Biochem. and Biophys. Res. Comm., 508, 1221-1226 (2019)).

RNA elements like Internal Ribosome Entry Sites (IRES) and ribozymes play a crucial role in regulating gene expression at the post-transcriptional level. Their functions, however, are quite distinct. Internal Ribosome Entry Sites (IRES) are RNA sequences found within the 5' untranslated region (UTR) of certain mRNAs. They enable a unique form of translation initiation that is cap-independent, allowing the ribosome to bind directly to the mRNA and start protein synthesis without the need for certain initiation factors typically required in cap-dependent translation. This method is naturally used by certain viruses to ensure their proteins are produced even when the host cell's cap-dependent translation is shut down and has been harnessed as a convenient biotechnological tool.

Ribozymes are RNA molecules that possess catalytic activity, similar to protein enzymes that catalyze specific biochemical reactions, including self-splicing and the cleavage and ligation of other RNA molecules. Ribozymes can influence gene expression by modifying RNA molecules post-transcriptionally, affecting their stability, processing, and translational efficiency. For instance, the Hepatitis Delta Virus (HDV) ribozyme and the Hammerhead ribozyme are often used in genetic engineering to precisely cleave RNA molecules at desired locations.

RNA-based switches, or RNA devices, are increasingly used in biotechnology to control gene expression in eukaryotic cells. These switches exploit the diverse functionality of RNA, from its ability to bind small molecules and proteins, to its capacity to fold into complex structures and undergo conformational changes. In the context of eukaryotic expression systems, they are particularly valuable due to their potential for precise and tuneable control over gene expression. For example, engineered riboswitches can be used to control gene expression in response to specific cellular or environmental conditions in eukaryotic cells. Riboswitches are naturally occurring elements in the non-coding regions of mRNA that can directly bind small molecules and undergo conformational changes, modulating transcription, translation, or mRNA splicing. Further, Toehold switches are a type of synthetic riboregulators developed for use in eukaryotic cells, which work by sequestering the ribosome binding site In a stable hairpin structure that prevents translation initiation. The switch is activated by the binding of a trigger RNA that unfolds the hairpin and frees the ribosome binding site. For enhanced induction of transgene expression in mammalian cells, "eToeholds" have been developed (ZHAO, EM et al., RNA-responsive elements for eukaryotic translational control, Nature Biotechnology, 40, 539-545 (2022)). These different types of RNA-based switches are often used in combination and integrated into synthetic genetic circuits to achieve complex behaviors, such as oscillations, feedback control, and conditional responses. They have potential applications in many areas of biotechnology, including metabolic engineering, cell-based therapies, biosensing, and gene therapy.

2A peptides can separate a longer polypeptide chain into two shorter ones. This strategy becomes particularly useful when a fused protein is non-functional. By integrating the coding sequence (CDS) of a 2A peptide at the fusion point, or by substituting a linker sequence with the CDS of a 2A peptide, the fused protein can be effectively split into two distinct polypeptides, thereby restoring their functionality. Moreover, 2A peptides can facilitate the production of multiple, distinct peptides from a single transcript. This enables the precise manipulation of protein expression, making 2A peptides an essential tool in biotechnological applications (LIU, Z, Systematic comparison of 2A peptides for cloning multi-genes in a polycistronic vector, Scientific Reports, 7, 2193 (2017)).

In a second aspect, there is provided a vector, or more than one, preferably two vectors, wherein the one or more vector(s) comprise(s) at least one nucleic acid molecule encoding the self-amplifying biologic according to the first aspect or embodiments thereof, wherein said vector(s) comprise(s) at least one nucleic acid molecule encoding the chimeric F-protein andH- protein as defined in the first aspect disclosed herein, and wherein said self-amplifying biologic assembles itself inside a biological host.

A vector refers to a vehicle or carrier that transfers genetic material from one organism to another. These vectors are typically DNA or RNA molecules such as plasmids or viruses. Vectors are widely used in genetic engineering and biotechnology for a variety of purposes, e.g. to carry foreign DNA into a host organism, where this genetic material can then be replicated, transcribed, and translated into protein. Key features of vectors often include a cloning site where the foreign DNA can be inserted, a marker gene to help identify successfully transformed cells, and, in many cases, a promoter sequence to initiate transcription of the inserted gene. For instance, plasmid vectors are small, circular pieces of DNA that can replicate independently of the host's chromosomal DNA. They are often used in bacterial cells for cloning, creating gene libraries, or producing proteins. Viral vectors, on the other hand, exploit the natural ability of viruses to infect cells and integrate their genetic material.

In another embodiment of the various aspects of the present invention, the nucleic acid molecules encoding the at least one protein according to the first aspect and the embodiments thereof, may be arranged in any combination with each other.

In further embodiments of the various aspects of the present invention, said vector(s) can be transferred into a cell via any suitable transfection protocol known in the art. Common transfection protocols for different types of vectors are known in the art.

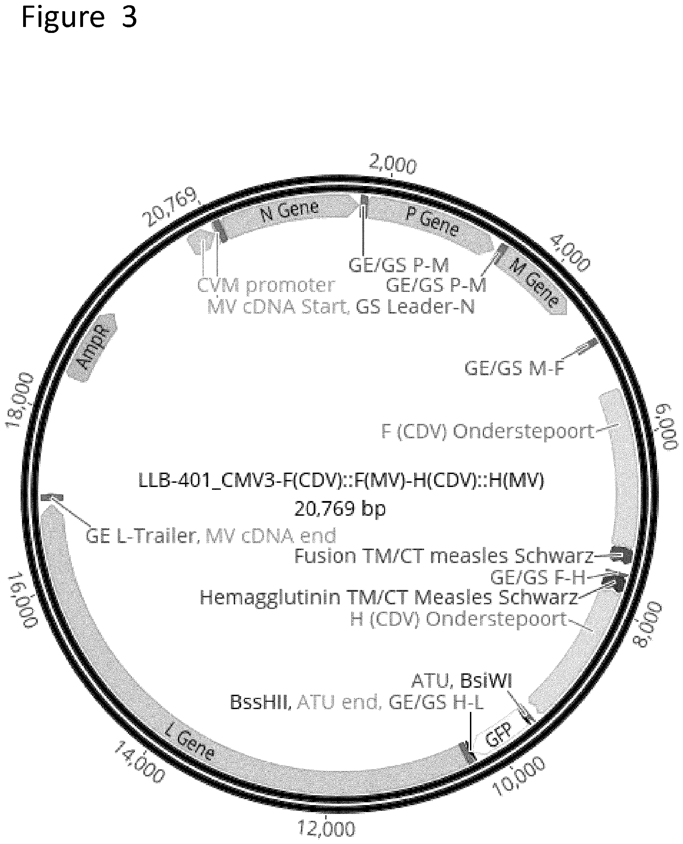

In one embodiment, as illustrated in FIGURE 3, one vector contains various genes, from the 5' end to the 3' end. A gene consists of an open reading frame (ORF) and upstream regulatory sequence denoted "GS" sequence and a downstream regulatory sequence denoted "GE" sequence. Upstream of the genes lies a CMV promoter with a nucleic acid sequence as specified by SEQ ID NO: 7. These gene transcription units include: (a) a nucleic acid molecule that encodes the MV's N-protein, (b) a nucleic acid molecule that encodes the MV's P-protein, (c) a nucleic acid molecule encoding the MV's M-protein, (d) a nucleic acid molecule encoding the chimeric CDV-MV F protein, (e) a nucleic acid molecule encoding the chimeric MV-CDV H protein, (f) an ATU encoding for green fluorescent protein (GFP) as a payload and (g) a nucleic acid molecule encoding the MV's L protein. The GS and GE sequences of the ATU are specified by SEQ ID NOs: 8 and 9. These nucleic acid molecules are functionally connected and regulated by viral replication and transcription sequences, such as the MV leader and trailer sequences, as specified by SEQ ID Nos: 10 and 11, respectively.

A person skilled in the art can easily clone a nucleic acid and therefore functionally insert a nucleic acid sequence encoding a payload of interest into said vector. In this context, a functional insertion means an introduction that permits the transcription and translation of all nucleic acid sequences encoded by the vector. This means that the insertion should not interfere with a regulatory sequence, such as a promoter or in any way that would compromise the structural and functional integrity of a protein encoded by said nucleic acid sequence , which includes structurally and functionally significant proteins of the respective paramyxovirus-namely, the "N-protein", "P-protein", "M-protein", "F-protein", "H-protein", and "L-protein".

The SelfAmp's assembly usually will occur in a host cell's cytoplasm. In certain embodiments, proteins and nucleic acid molecules of a SelfAmp are provided separately, wherein the proteins are expressed and purified separately and nucleic acid molecules are amplified in a separate step, e.g. by PCR, but not limited thereto. Subsequent assembly of the SelfAmp can take place within a cell, or outside a cell, in vivo or in vitro, preferably in vitro under controlled GMP conditions.

A biological host is a cell or organism that is used as a recipient for foreign biological or synthetic material, including a SelfAmp, often in the form of nucleic acid molecules or proteins, but not limited thereto. The host can be a single cell, like a bacterium, a fungus, a protozoan, a single cell originating from an animal or a plant, or a multi-cellular organism like a plant, a fungus or an animal. When the host organism takes up the foreign material, the host's cellular machinery is used to replicate the foreign nucleic acid molecules and/or expresses the proteins encoded by that nucleic acid molecules e.g., in the context of viral therapy, the host is the patient's own cells.

Further in one embodiment of the second aspect disclosed herein, there is provided at least one vector according to the second aspect, characterized in that the at least one nucleic acid molecule as disclosed in the second aspect, comprises a chimeric sequence of SEQ ID NOs: 1 and 2, or 12, or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any of the SEQ ID NOs: 1 and 2, or 12, respectively.

In another embodiment, there is provided at least one vector according the second aspect and the embodiment disclosed therein, characterized in that the at least one nucleic acid molecule, as disclosed in the second aspect and previous embodiment, additionally comprises a sequence of SEQ ID NOs: 3, 4, 5 and 6 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any of the SEQ ID NOs: 3, 4, 5 and 6 respectively.

In a third aspect, there is provided a method of producing a self-amplifying biologic of the first and second aspect and the embodiments disclosed therein, the method comprising: (i) providing the at least one self-amplifying biologic as defined in the first aspect and the embodiments disclosed therein, and/or providing the at least one vector according to the second aspect and the embodiments disclosed therein, and introducing said at least one self-amplifying biologic and/or said at least one vector into at least one host cell, (ii) culturing the at least one host cell under conditions suitable for amplification of said at least one self-amplifying biologic and/or said at least one vector, (iii) allowing the assembly of at least one self-amplifying biologic from the at least one vector; and (iv) optionally: purifying said at least one self-amplifying biologic of step (iii); and (v) obtaining the at least one self-amplifying biologic.

The production of SelfAmps in cell culture is a meticulous process and follows usual protocols known in the art for the purification of viruses: This includes the selection of the appropriate cell line, depending on the specific type of virus to be produced, including Vero cells, HEK293 cells, or any other suitable human, mammalian or insect cell line, as helper cells for virus replication. After the selection of the appropriate cell line, the cells are cultured in a controlled environment, often in a flask or a bioreactor. The cells are sustained in a medium that provides all necessary nutrients and thus suitable conditions for their growth, including sugars, amino acids, vitamins, and growth factors according to established protocols depending on the cell type. Once the cells have reached the desired density, they are transfected with the virus by standard procedures known in the art. The virus infects the cells and employs the cell's machinery for its replication, whereby the incubation period varies depending on the specific virus. After the virus replication cycle is completed, the newly formed virus particles are released from the cells either through cell lysis or via a budding process, where the virus particles are released via the cell membrane, leading to virus accumulation in the culture medium.

In certain embodiments of the aspects of the present invention, the host cell is cultured after inoculation with a self-amplifying biologic.

Further, in certain embodiments, the host cell is cultured after transfection with the at least one vector encoding the self-amplifying biologic.

In certain embodiments, the host cells containing the self-amplifying biologic are stored under suitable conditions known in the art.

In one embodiment according to certain aspects of the present invention, a purification step leads to SelfAmps with a lower concentration of process-related impurities. These impurities might include, but are not limited to, host cells, cell debris, protein contaminants stemming from cell culture additives or enzymes used during cultivation and processing, a microcarrier used for host-cell cultivation, or foreign nucleic acids not related to either the host cell or the recombinant infectious virus or its particles of interest.

The method can include an incubation step where the infected host cell is kept at a temperature within the range of 32 to 37°C. From the cell culture medium, a sample may be taken, which represents SelfAmp particles as expressed and released from the host cell. This sample may comprise infectious virus particles and/or virus-like particles and/or recombinant subviral particles.

In this context, a "sample" refers to a sample collected from an infected host cell culture during the various steps of the methods of the present application. It could be a sample obtained from the supernatant or the lysate of a cell. The sample can be used for further clarification and/or purification, or for analysis, such as determining the correct sequence of a transcribed virus genome or analyzing the SelfAmp with methods known in the art and usually applied for purified viruses.

In embodiments utilizing purification, the purified SelfAmps are usually obtained in one or more fractions corresponding to the product peak of the chromatography elution step, dependent on the retention volume of the SelfAmp.

In another embodiment, there is provided a method according to the third aspect, further comprising a step of (vi) introducing or contacting said at least one self-amplifying biologic as obtained in step (v) of the third aspect into at least one target cell or in vitro cellular system for transfecting said at least one target cell and/or for studying the functionality of the at least one self-amplifying biologic.

In certain embodiments, an in vitro cellular system refers to any experimental setup where living cells are studied outside of their normal biological context, typically in a controlled laboratory environment. In these systems, cells can be derived from various tissues or organs of various organisms, including humans, and are often maintained under specific conditions, e.g., temperature, humidity and/or nutrient supply that mimic their natural environment as closely as possible. The cells can be of a single type (monoculture) or multiple types (co-culture).

These systems provide a platform for understanding fundamental biological processes at the cellular level, such as cell growth, division, differentiation, and response to various stimuli e.g., drugs, toxins or hormones, preferably after introduction of the SelfAmp into the cells and/or the cell culture.

In a fourth aspect, there is provided a use of the at least one self-amplifying biologic according to any of the previous aspects and embodiments as a diagnostic tool, and/or for the introduction of at least one reporter molecule or a reporter system into a cell and/or for recombinant protein expression in cell culture, such as cytotoxic protein expression and/or production of at least one co-factor for virus production, and/or for circulating cancer cell diagnosis and/or for the generation of any modified cell, preferably a CAR-T or a CAR-NK cell, and/or for ex vivo gene therapy and/or ex vivo gene transfer.

In certain embodiments, said reporter molecule or reporter system comprises fluorescent proteins such as, Green Fluorescent Protein (GFP), Cyan Fluorescent Protein (CFP), Yellow Fluorescent Protein (YFP) or Red Fluorescent Protein (RFP), luciferases, such as firefly luciferase or Renilla luciferase, beta-galactosidase, alkaline phosphatase, horseradish peroxidase (HRP), chloramphenicol acetyltransferase (CAT) or aequorin, but not limited thereto.

In certain embodiments the self-amplifying biologic may be used for recombinant protein expression, whereas recombinant protein expression is the production of specific proteins by harnessing the protein synthesis machinery of a host organism. The process begins with gene cloning, where the gene encoding the protein of interest is isolated and incorporated into a vector, resulting in a recombinant nucleic acid molecule. This recombinant nucleic acid molecule is then introduced into a suitable host organism. Once inside the host, the inserted gene serves as a template for the synthesis of the desired protein. The host organism's cellular machinery facilitates this process, translating the genetic code into a functional protein. Following protein expression, the resultant protein is then isolated from the host organism. Alternatively, protein expression may be performed in an in vitro expression system. Through a series of purification steps by methods known in the art, the desired protein is separated from other proteins and impurities, resulting in a highly purified protein product.

In certain embodiments, a self-amplifying biologic may be used in circulating cancer cell diagnosis, whereas circulating cancer cell diagnosis refers to the detection and analysis of circulating tumor cells (CTCs). These are cancer cells that have detached from a primary tumor and circulate in the bloodstream. The presence of CTCs is a common phenomenon in many types of cancer and a crucial factor in the process of metastasis, where cancer spreads to distant organs.

Chimeric Antigen Receptor T (CAR-T) and Chimeric Antigen Receptor Natural Killer (CAR-NK) cells represent sophisticated approaches within the field of cellular immunotherapies, aimed at harnessing the patient's own immune system to combat malignant tumors. CAR-T cell therapy involves the genetic engineering of a patient's autologous T lymphocytes to express a chimeric antigen receptor (CAR) that targets a specific antigen expressed on the surface of malignant cells. Following ex vivo expansion, these modified T cells are then reintroduced into the patient, where they proceed to bind the target antigen, thereby initiating an immune response against the cancer cells. CAR-T therapy has been particularly efficacious in hematological malignancies such as B-cell lymphomas and acute lymphoblastic leukemia, with substantial rates of remission observed. CAR-NK therapy, follows a similar principle of cellular engineering. Natural Killer (NK) cells, known for their inherent cytotoxic capabilities against a broad spectrum of target cells, are modified to express a CAR for a specific cancer antigen. Relative to CAR-T cells, CAR-NK cells have the potential advantage of reducing certain adverse events associated with CAR-T therapies, such as cytokine release syndrome and graft-versus-host disease.

Ex vivo gene therapy is a procedure, where cells are removed from a patient's body and genetically modified in a laboratory to correct a genetic defect or to confer a new function. The process typically involves using viral vectors or may involve other delivery methods, e.g., via SelfAmps, to introduce a healthy copy of a gene into the patient's cells to replace or compensate for a faulty one. The cells are then cultured and multiplied in the lab to create a sufficient number of them for therapeutic use. Once the cells have been genetically modified and expanded, they are reintroduced into the patient's body. Here, they should begin to carry out the function of the faulty gene, thus alleviating the symptoms of the disease. Ex vivo gene transfer follows an analogous process of introducing a new gene into the cells outside of the body (ex vivo) before they are returned to the patient.

In a fifth aspect, there is provided a pharmaceutical composition comprising the at least one self-amplifying biologic according to the first aspect and embodiments thereof.

In another aspect, there is provided a pharmaceutical composition comprising the at least one self-amplifying biologic obtained by or obtainable by a method according to the third aspect and the first embodiment thereof, and optionally, at least one pharmaceutically acceptable carrier or excipient.

In certain embodiments, the pharmaceutically acceptable carrier or excipient comprises at least one bulking agent and/or at least one binder and/or at least one disintegrant and/or at least one coating and/or at least one filler or diluent and/or at least one stabilizer or preservative and/or at least one solvent and/or at least one solubilizer.

In one embodiment, there is provided a pharmaceutical composition according to the previous aspect for use in a method of treating or preventing a disease.

In certain embodiments, said disease may be a chronic infectious disease, such as HIV/AIDS, Tuberculosis (TB), Hepatitis A, Hepatitis B, Hepatitis C, Human papillomavirus (HPV) infection, Herpes simplex virus (HSV) infection, Epstein Barr virus (EBV) infection, Lyme disease, Chagas disease, Malaria or Schistosomiasis, but not limited thereto.

In certain embodiments, said disease may be a cancer, such as bladder cancer, breast cancer, colon cancer, rectal cancer, endometrial cancer, kidney cancer, leukemia, liver cancer, lung cancer, melanoma, non-Hodgkin lymphoma, pancreatic cancer, prostate cancer or thyroid cancer, but not limited thereto. An even more comprehensive list of eligible types of cancer is published by the NIH National Cancer Institute (https://www.cancer.gov/types).

In other embodiments, said disease may be a metabolic disease or and/or a genetic disorder.

In another embodiment, there is provided a pharmaceutical composition according to the previous aspect for use in a method for the treatment and/or prevention of an inflammatory disease, preferably an autoimmune disease, or a cancer.

In another embodiment, the inflammatory disease may be any autoimmune disease selected from the "Autoimmune Disease List" of the Global Autoimmune Institute (https://www.autoimmuneinstitute.org/resources/autoimmune-disease-list/), but not limited thereto.

An inflammatory disease is accompanied by specific symptoms related to inflammation, as well as the presence of certain biomarkers, which can be detected through medical testing. Inflammatory diseases often present common symptoms such as redness, swelling, heat, and pain in the affected area, as well as systemic symptoms like fatigue, weight loss, and fever as clinical signs. Blood tests can reveal specific biomarkers that suggest inflammation, e.g., a high level of C-Reactive protein (CRP), a high Erythrocyte Sedimentation Rate (ESR), high levels of white blood cells as measured in a Complete Blood Count (CBC) and/or upregulation of certain cytokines, such as interleukin-1 (IL-1), interleukin-6 (IL-6), and/or tumor necrosis factor alpha (TNF-α), but not limited thereto. Further, medical imaging tests can often reveal inflammation in specific organs or tissues. For example, in rheumatoid arthritis, X-rays may reveal joint damage, while in inflammatory bowel disease, a colonoscopy or other imaging studies can reveal inflammation in the intestines.

In a further embodiment, there is provided a pharmaceutical composition according to the previous embodiment, wherein the inflammatory disease is selected from the group consisting of Multiple Sclerosis, Rheumatoid Arthritis, Gout, Psoriasis, Asthma, Crohn's Disease, Chronic Obstructive Pulmonary Disease, Allergies, Hepatitis, Dermatitis or Sarcoidosis.

In certain embodiments, said pharmaceutical composition is one component of a combination therapy, whereas combination therapy refers to the use of more than one medication or treatment modality for a single disease or condition. Combination therapy can have advantages of enhanced therapeutic efficacy by targeting multiple aspects of a disease or condition, reducing the likelihood of resistance and/or reducing side effects. The pharmaceutical composition can be used in combination with all known medications, such as small molecules or biologics, or with treatment modalities known in the art, such as antibody therapy, chemotherapy, radiation therapy, immunotherapy, hormone therapy or gene therapy, but not limited thereto.

In a further embodiment, there is provided a kit comprising the self-amplifying biologic according to the first aspect and the embodiments disclosed therein, and/or a vector according to the second aspect and the embodiments disclosed therein, optionally wherein the kit comprises further reagents, including at least one buffer or solution.

The kit will usually contain all ingredients, with or without suitable expression host cells that allow the amplification and/or assembly of a functional self-amplifying biologic.

Further aspects and embodiments of the present invention can be derived from the subsequent, non-limiting, detailed description, the Figures, the Sequence Listing, as well as the attached set of claims.

### Examples

The present invention is further illustrated by the following non-limiting examples.

### Example 1: Design and Cloning of a vector coding for a Self Amp

Canine morbillivirus (former Canine distemper virus) belongs to the family Paramyxoviridae and is closely related to Measles Virus. 17 different lineages are known. The virus causes a highly contagious infection in Canoidea, but is apathogenic in humans. Different live-attenuated vaccine strains exist - among these are the Onderstepoort Strains OS and OL which differentiate in their plaque size. Both strains were generated through multiple cell culture passages.

The CDV virions have an envelope in which the glycoproteins hemagglutinin H and fusion F are embedded. These proteins are responsible for receptor binding and fusion of the viral membrane with the cellular membrane. The CDV glycoproteins were chosen to be inserted into a measles vector to circumvent pre-existing immunity. The sequences of the H and F glycoproteins of the CDV Onderstepoort Strain were selected from the NCBI database and synthesized. Next, the extracellular domain of CDV H and F were in silico combined with the transmembrane domain and the cytoplasmic domain of the measles Schwarz H and F proteins, respectively. Measles Schwarz vector served as template for the measles specific sequences. Primers were designed to fuse the extracellular domains of CDV H and F to the transmembrane and intracellular measles domains via PCR reaction. Three different PCRs were generated covering the in silico designed chimeric sequences. PCR bands corresponding to the correct size were gel purified and subjected to Gibson Assembly together with Sfil-digested measles Schwarz vector backbone.

To create a bisegmented SelfAmp, certain parts of the measles virus genome were amplified via PCR using designed primers. The PCR bands were gel purified and subjected to Gibson Assembly. Both genome segments contain a leader sequence and a trailer sequence at the 3 and 5 end, respectively, to allow for efficient replication.

### Example 2: Self-Amp amplification and purification

Plasmids containing a leader sequence, measles genes and a trailer sequence were introduced into helper cells via transfection. Together with these plasmids additional helper plasmids were co-transfected. Helper plasmids contain measles genes that are expressed in mammalian cells upon transfection - N, P and L proteins of MV virus. In addition, plasmids containing F and H genes of MV virus or chimeric F and H genes could also be transfected together with the other plasmids to promote initial virus production and reduce the selection pressure.

All transfectable cells can be helper cells. Here, HEK 293T cells were used.

After transfection, cells were co-cultured with passaged cells suitable for the passage of MV attenuated strains. Co-cultures were harvested and transferred onto freshly seeded Vero or Huh-7 cells. Plates on which syncytia formed were harvested and the viral titerwas determined via TCID50, as described in Example 4. Payload expression was confirmed via fluorescent based immunofocus assay, as described in Example 3.

Recombinant MV was purified using standard purification methods to remove large particles and small molecule impurities. The skilled person will well be aware of suitable purification strategies from protocols available in the art, for example, as described in NESTOLA, P et al., Improved virus purification processes for vaccines and gene therapy, Biotechnology and Bioengineering, 112, 5, 843-857 (2015), Vicente, T et al., Large-scale production and purification of VLP-based vaccines, Journal of Invertebrate Pathology, 107, 542-548 (2011) or EP 3184118 B1.

### Example 3: Analysis of syncytia formation

Vero cells were infected with recombinant MV and incubated for three days to allow syncytia formation. To avoid extensive virus spread, cells were overlaid with an Avicel/CMC mix. To evaluate syncytia formation, cells were fixed and permeabilized and then stained with an MV-NP antibody and an appropriate fluorescence labeled secondary antibody. A potential payload could be co-stained as well. Syncytia formation was detected with an automated microscope (ImageXpress^{®} Pico Automated Cell Imaging System).

### Example 4: Viral titer determination

To determine viral titer, Vero cells were infected with a serial dilution of virus in eight replicates. After a seven-day incubation period, infected wells were observed under the microscope and virus titers were calculated using the Spearman/Käber method.

### Example 5: Evaluation of cell growth characteristics

To evaluate cell growth characteristics of recombinant MV/self-Amp, Vero cells were infected with an MOI of 0.01 with the appropriate samples. Supernatant (SN) as well as cells were harvested after day 1, 2, 3, 4, 5 and 6 and frozen at -80°C. RNA copies were determined by isolating RNA from all conditions (SN and CL; cell lysates) and analyzed via qPCR using appropriate primers covering an intergenic region of the SelfAmp. In addition, viral titer was assessed via TCID50 according to example #4. RNA copies per infectious particle can then be calculated from the previously obtained values.

### Example 6: Evaluation of immune-neutralization efficiency

Recombinant MV/Self-Amp was preincubated with goat anti-MV serum in different dilutions for 1h at room temperature. Then, Vero cells were infected with virus as well as with virus pre-incubated with serum and left for 3 days with Avicel/CMC mix overlay. Immune-neutralization efficiency was evaluated by fixation/permeabilization of Vero cells and staining for MV-nucleoprotein as well as for cell nucleic acid (DAPI). Analysis of virus infected cells (positive cells for NP) were calculated, and neutralization efficiency of Self-Amp were determined.

## Claims

1. A self-amplifying biologic comprising or consisting of:
(i) at least one N-protein, at least one P-protein, at least one accessory protein C, at least one accessory protein V, at least one M-protein and at least one L-protein, and/or a sequence encoding the same, wherein the at least one protein originates from at least one Paramyxovirus, preferably from a measles virus; and
(ii) at least one F-protein and/or at least one H-protein originating from the same paramyxovirus of (i), wherein the at least one F-protein and/or at least one H-protein each comprise an ectodomain and a transmembrane domain, wherein said ectodomain is substituted against an ectodomain of at least one different paramyxovirus in comparison to the paramyxovirus of (i).

2. The self-amplifying biologic according to claim 1, wherein the self-amplifying biologic further comprises at least one payload, and/or a sequence encoding the same.

3. The self-amplifying biologic according to any one of the preceding claims, wherein a target moiety is fused to the H-protein, preferably to the H-protein C-terminus or to a loop region of the H-protein, that binds to a target receptor associated with the host cell and thus targets the self-amplifying biologic to a receptor expressing cell, wherein the target moiety is a binding protein or a sequence encoding the same, comprising a natural ligand or an antibody or antibody fragment, comprising a scFv, or any peptide, comprising a DARPin or a nanobody, or any molecule that interacts with a target receptor associated with the host cell.

4. The self-amplifying biologic according to claims 2 and 3, wherein the payload is encoded as an additional transcription unit and/or is expressed under the control of at least one regulatory RNA element, preferably an IRES or a ribozyme, or a cell type-specific or cell state-specific RNA-based switch and/or further modified via a self-cleaving peptide, preferably a 2A-peptide, or a combination thereof.

5. A vector, or more than one, preferably two vectors, wherein the one or more vector(s) comprise(s) at least one nucleic acid molecule encoding the self-amplifying biologic according to any one of the preceding claims, wherein said vector(s) comprise(s) a at least one nucleic acid molecule encoding the chimeric F- protein and H-protein as defined in claim 1, and wherein said self-amplifying biologic self-replicates and self-assembles itself inside a biological host.

6. The at least one vector according to claim 5, **characterized in that** the at least one nucleic acid molecule comprises a chimeric sequence of SEQ ID NOs: 1 and 2, or 12, or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any of the SEQ ID NOs: 1 and 2, or 12, respectively.

7. The at least one vector according to claim 6, **characterized in that** the at least one nucleic acid molecule additionally comprises a sequence of SEQ ID NOs: 3, 4, 5 and 6 a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any of the SEQ ID NOs: 3, 4, 5 and 6 respectively.

8. A method of producing the self-amplifying biologic according to claims 1 to 4, the method comprising:
(i) providing the at least one self-amplifying biologic as defined in claim 1 to 4 and/or providing the at least one vector according to claims 5 to 7, and introducing said at least one self-amplifying biologic and/or said at least one vector into a host cell,
(ii) culturing the at least one host cell under conditions suitable for amplification of said at least one self-amplifying biologic and/or said at least one vector,
(iii) allowing the assembly of the at least one self-amplifying biologic from the at least one vector; and
(iv) optionally: purifying said at least one self-amplifying biologic of step (iii); and
(v) obtaining at the least one self-amplifying biologic.

9. The method of claim 8 further comprising a step of:
(vi) introducing or contacting said at least one self-amplifying biologic as obtained in step (v) of claim 8 into at least one target cell or in vitro cellular system for transfecting said at least one target cell and/or for studying the functionality of the at least one self-amplifying biologic.

10. A use of the at least one self-amplifying biologic according to any of the preceding claims as a diagnostic tool, and/or for the introduction of at least one reporter molecule or a reporter system into a cell and/or for recombinant protein expression in cell culture, such as cytotoxic protein expression and/or production of at least one co-factor for virus production, and/or for circulating cancer cell diagnosis and/or for the generation of any modified cell, preferably a CAR-T or a CAR-NK cell, and/or for ex vivo gene therapy and/or ex vivo gene transfer.

11. A pharmaceutical composition comprising the at least one self-amplifying biologic according to any one of claims 1 to 4, or a pharmaceutical composition comprising the at least one self-amplifying biologic obtained by or obtainable by a method according to claim 8 or 9, and optionally, at least one pharmaceutically acceptable carrier or excipient.

12. The pharmaceutical composition according to claim 11 for use in a method of treating and/or preventing a disease.

13. The pharmaceutical composition according to claim 11 for use in a method for the treatment and/or prevention of a disease, wherein the disease is independently selected from an inflammatory disease, preferably an autoimmune disease, or a cancer.

14. The pharmaceutical composition for use in a method for the treatment and/or prevention of a disease according to claim 13, wherein the inflammatory disease is selected from the group consisting of Multiple Sclerosis, Rheumatoid Arthritis, Gout, Psoriasis, Asthma, Crohn's Disease, Chronic Obstructive Pulmonary Disease, Allergies, Hepatitis, Dermatitis or Sarcoidosis.

15. A kit comprising the self-amplifying biologic according to claims 1 to 4, and/or a vector according to claims 5 to 7, optionally wherein the kit comprises further reagents, including at least one buffer or solution.
